# EUROPEAN PATENT APPLICATION

(11) **EP 1 875 885 A1**
(43) Date of publication of application: **09.01.2008**
(21) Application number: 06014038.1
(22) Date of filing: 06.07.2006
(51) Int. Cl.: A61F 5/449

(54) **A method of providing an ostomy support garment**

(71) Applicant: Vayani Enterprises Limited, Blaby Leicester LE9 4GZ (GB)
(72) Inventor: Vayani, Razak, Leicester, LE5 6QA (GB)
(74) Representative: Lucking, David John

(57) **Abstract**

A method of providing an ostomy support garment 12 with an opening 10 for receiving a stoma of a particular ostimate 15 includes providing a model garment 16, the model garment 16 being of substantially the same size and configuration as the ostomy support garment 12, and the model garment 16 including a marked region 17 which when the model garment 16 is worn by the ostimate 15, overlies the stoma, the marked region 17 including a plurality of areas each individually marked with a unique marking to identify the area, and, with the ostimate 15 wearing the model garment 16 so that the marked region 17 overlies the stoma, the method including recording the unique marking of the or each area which overlies the stoma, to provide data, and subsequently using the data for manufacturing the ostomy support garment 12 with the opening 10 in a position appropriate for receiving the particular ostimate's 15 stoma.

## Description

This invention relates to a method of providing an ostomy support garment for an ostimate.

It is known from W02004/069115 to provide a model garment of the same size and shape as the ostomy support garment it is desired to produce, which model garment is worn by an ostimate to locate, with respect to a grid marked on the model garment, co-ordinates of the centre of the ostimate's stoma where a corresponding opening is required in the ostomy support garment. These co-ordinates are then used to produce ostomy garments with openings in the correct positions for the particular ostimate.

Such a method is not straightforward to perform, requiring a stoma nurse for an example of a person required to locate the position of the centre of the stoma, to have particular skill to decide on the co-ordinates where on the grid, the centre of the stoma is. To help identify the centre of the stoma, the known method uses an opening locating device which is placed over the stoma beneath the model garment, the opening locating device having a single central protuberance. The stoma nurse then has to make an assessment of the co-ordinates of the protuberance on the grid and record these.

Not only is it difficult to assess the correct co-ordinates of the centre of the stoma even using the opening locating device, but because stomas are of all different shapes and sizes, merely knowing where the centre of the stoma is, according to the opening locating device, does not ensure that the opening which is provided in the stoma support garment is in the correct position or of the correct size.

According to one aspect of the present invention we provide a method of providing an ostomy support garment with an opening for receiving a stoma of a particular ostimate, the method including providing a model garment, the model garment being of substantially the same size and configuration as the support garment, and the model garment including a marked region which when the model garment is worn by the ostimate, overlies the stoma, the marked region including a plurality of areas each individually marked with a unique marking to identify the area, and, with the ostimate wearing the model garment so that the marked region overlies the stoma, the method including recording the unique marking of the or each area which overlies the stoma, to provide data, and subsequently using the data for manufacturing the ostomy support garment with the opening in a position appropriate for receiving the particular ostimate's stoma.

Thus using the method of the invention, a stoma nurse or other person may locate the optimum position for the opening in the ostomy support garment to be produced, and does not have to make a skilled assessment of co-ordinates on any grid, but merely needs to record the unique marking of the or each area which overlies the stoma. Thus the method also enables the size of the opening in the support garment to be produced, to be determined, as this will depend on the number of individually marked areas which overlie the stoma.

The areas which are uniquely marked may be polygons. The polygons may all be the same and of the same size. Preferably the polygons are contiguous i.e. the polygons nest so that there are no spaces between polygons. Thus most conveniently the polygons are hexagons.

Each hexagon or other polygon would be uniquely marked with, for example, a colour and/or a character such as one or more numbers or text characters.

In a preferred embodiment, the method includes providing an opening locating device which is locatable around the stoma, and receiving the stoma in a receiving part of the opening locating device.

The device may include one continuous edge or a plurality of separated protuberances which when the device is located over the stoma, surround the stoma, the method including, when the model garment is worn by the ostimate, recording the unique marking of the or each area of the marked region overlying the edge or protuberances, so that the data provided indicates the boundary of the opening to be provided in the support garment rather than just the centre of the opening for the stoma. This provides for a more reliable indication of the desired opening position in the ostomy support garment than methods which only record an approximate centre of the opening.

If desired, the method may include connecting the opening locating device to the ostimate around the stoma whilst determining the marked area or areas which overlie the stoma.

It will be appreciated that an ostomy container (or bag) typically is attached to the ostimate by an annular flange which surrounds the stoma. The opening locating device may be sized generally to correspond to such a flange, and thus the method may include selecting an opening locating device of a size corresponding to such a flange, which will be dependent upon the size of the stoma. Thus the opening size in the ostomy support garment may be made suitable to receive and support a part of an ostomy container which in use is secured to the flange through the opening in the support garment.

According to a second aspect of the invention we provide an opening locating device for locating an optimum position for an opening in an ostomy support garment on a model garment, the opening locating device including an annular peripheral formation generally corresponding in size to that of a flange to which in use an ostomy container is secured, and the device including a receiving part bounded by the peripheral formation, for receiving an ostimate's stoma, the device including a continuous edge or a plurality of protuberances which when the device is located over the flange and the ostimate is wearing the model garment, is or are positioned around a boundary of the stoma, and indicate on the model garment the position of a boundary of the opening to be provided in the ostomy support garment.

Embodiments of the invention will now be described with the aid of the accompanying drawings in which:-
FIGURE 1 is an illustrative view of a model garment for use in locating the position of an opening to be provided in an ostomy support garment;
FIGURE 2 is an illustrative view of an opening locating device for use with the model garment of figure 1;
FIGURE 3 is a view similar to figure 1 but of the ostomy support garment supporting an ostomy container.

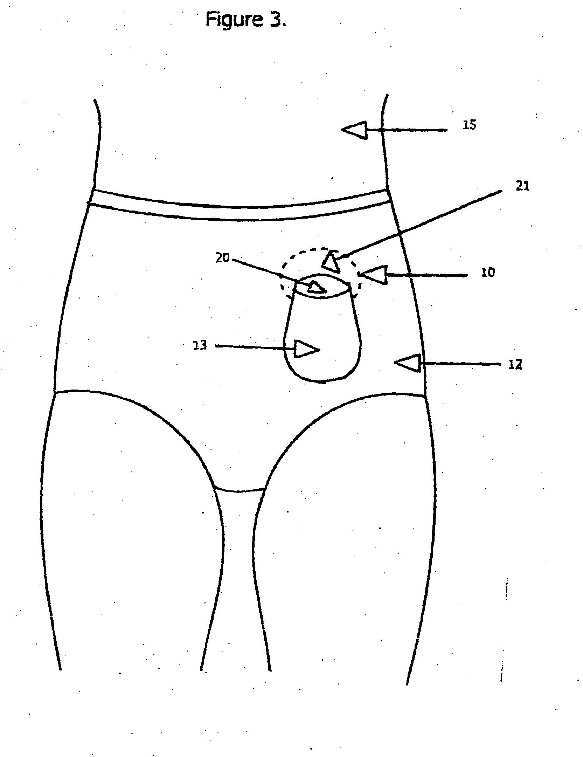

Referring to figure 3 of the drawings, to provide an opening 10 in an ostomy support garment 12 in an optimum position to support an ostomy bag 13 or other ostomy container, the following method is employed.

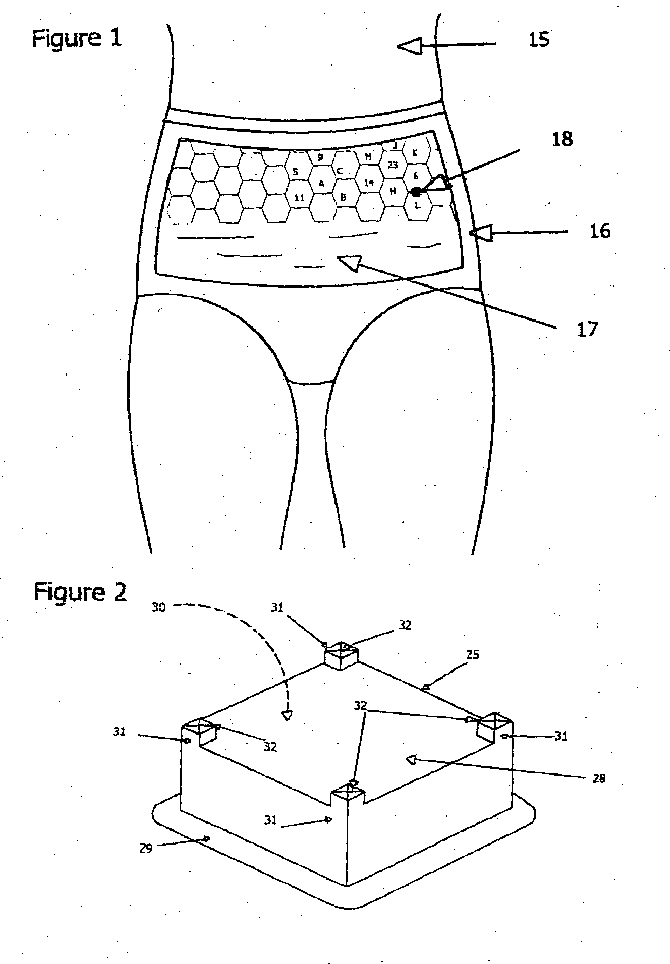

The ostimate 15 puts on a model garment indicated in figure 1 at 16. The model garment 16 is of the same size and configuration as the ostomy support garment 12, and preferably is made of the same material. Provided on a front panel of the model garment 16 is a marked region 17 which overlies at least the most likely areas of the ostimate 15 where a stoma is provided. In figure 1, the position of the ostimate's stoma beneath the model garment 16, is indicated at reference 18.

The marked region 17 includes a plurality of individually and uniquely marked areas which thus identify the areas, with in this example, two of the areas indicated at "6" and "L" overlying the ostimate's stoma 18.

In this example, the markings of the marked areas are characters, either text characters or numbers, but could be any recognisable marking, which can readily be recorded as hereinafter described. The markings may be orderly with adjacent markings being in a progression of markings, or as indicated in the example of figure 1, the markings may be random.

In the example, the marked areas are hexagonal, and are arranged so that the marked areas are contiguous i.e. they nest and there are no spaces between them. Thus marked areas overlie the entire area of the ostimate 15 where a stoma may be provided.

The method further includes a stoma nurse or other person, recording the position of the ostimate's stoma 18 by referencing the marked areas, thus providing data that for the ostomy support garment 12, an opening 10 is required corresponding to the position 18 of the stoma, in the example referenced by "6" and "L".

The position 18 of the stoma does not simply indicate the centre of the stoma, but encompasses the boundary of the stoma. In another example, the stoma may lie beneath a single marked area, or as in the example, two marked areas but other than "6" and "L", or more than two areas. The stoma nurse records the unique markings for the or all of the marked areas beneath which the stoma extends.

The data thus provided may then be used by an ostomy support garment manufacturer or finisher, to provide the ostomy support garment 12 with an opening 10 in an appropriate place, and of an appropriate size, to receive a part 20 of the ostomy bag 13 through the opening 10, to support the ostomy bag 13 in use when it is connected to an annular or other hollow flange 21 attached to the ostimate 15 around the stoma.

The data recorded by the ostomy nurse or another person may be used to produce any desired number of ostomy support garments 12 personalised for the ostimate 15 by the opening 10 position.

It will be appreciate that the markings of the areas beneath which the stoma is positioned may be recorded manually or electronically, and that the data may be used manually or electronically by the ostomy support garment 12 manufacturer or finisher. Where used manually, the manufacturer or finisher may have a template for placing on an ostomy support garment 12, the template having marked areas corresponding to the marked region 17 of the model garment 16, so that the ostomy support garment 12 may be marked and then provided with the opening 10 in the appropriate position. More typically in an automated manufacturing/finishing process, a machine will be programmed with the template, and thus may make the opening 10 from the data in the appropriate position without first marking the position of the opening 10.

Referring now to figure 2, there is shown an opening locating device 25 for assisting determining which of the marked areas of the marked region 17 of the model garment 16 need to be recorded for most reliably ensuring that the opening 10 is optimally provided in the ostomy support garment 12.

The device typically is made from a light transparent plastics material.

An ostimate 15 typically has attached to his or her person, an annular or other hollow flange 21 for the purpose of connecting an ostomy bag 13 around the stoma. The opening locating device 25 includes a peripheral formation 29 of a size and configuration at least close to the flange 21. Where the peripheral formation and the flange 21 are of the same size and shape, the device 25 could be connected to the flange 21, but at least is positionable accurately over the flange 21 whilst the ostimate 15 is wearing the model garment 16 and while the marked areas beneath which the stoma lies, are recorded. If the ostimate 15 does not have a flange 21 attached around the stoma, the device 25 may simply be placed over the stoma.

The device 25 further includes a receiving part 30 inwardly of the peripheral formation 29, to receive the stoma. The receiving part 30 includes in this example, four protuberances 31, one at each corner, which thus define the boundary of the opening 10 to be provided in the ostomy support garment 12.

Each protuberance 31 includes a rounded cap 32 which may readily be identified beneath the model garment 16.

In another example, instead of a plurality of protuberances 31 arranged around the desired opening 10, a single continuous edge may be provided. In each case, the opening locating device 25 clearly indicates to the stoma nurse or other person recording the marked areas beneath which the stoma lies, the position of the boundary of the stoma and thus the desired peripheral extent of the opening 10 to be provided in the ostomy support garment 12.

Various other modifications may be made without departing from the scope of the invention. For example the configuration of the ostomy support garment 12 shown, and the corresponding model garment 16, and of the opening locating device 25 are purely exemplary.

The periphery of the opening 10 provided in the ostomy support garment 12 is preferably elasticated so as to support the part 20 of the ostomy bag 13 which extends through the opening 10, but in an alternative embodiment, some other arrangement for closing the opening 10 around the part 20 of the ostomy bag 13, for example a drawstring arrangement, may be provided.

Instead of the marked areas of the marked region 17 of the model garment 16 being hexagons, other shaped marked areas may be provided, preferably polygons which nest and be arranged contiguously so that no spaces are provided between adjacent polygons.

The features disclosed in the foregoing description, or the following claims, or the accompanying drawings, expressed in their specific forms or in terms of a means for performing the disclosed function, or a method or process for attaining the disclosed result, as appropriate, may, separately, or in any combination of such features, be utilised for realising the invention in diverse forms thereof.

## Claims

1. A method of providing an ostomy support garment with an opening for receiving a stoma of a particular ostimate, the method including providing a model garment, the model garment being of substantially the same size and configuration as the support garment, and the model garment including a marked region which when the model garment is worn by the ostimate, overlies the stoma, the marked region including a plurality of areas each individually marked with a unique marking to identify the area, and, with the ostimate wearing the model garment so that the marked region overlies the stoma, the method including recording the unique marking of the or each area which overlies the stoma, to provide data, and subsequently using the data for manufacturing the ostomy support garment with the opening in a position appropriate for receiving the particular ostimate's stoma.

2. A method according to claim 1 wherein the areas which are uniquely marked are polygons.

3. A method according to claim 2 wherein the polygons are all the same and of the same size

4. A method according to claim 2 or claim 3 wherein the polygons are contiguous.

5. A method according to any one of claims 2 to 4 wherein the polygons are hexagons.

6. A method according to any one of the preceding claims wherein the method includes providing an opening locating device which is locatable around the stoma, and receives the stoma in a receiving part of the opening locating device.

7. A method according to claim 6 wherein the opening locating device includes one continuous edge or a plurality of separated protuberances which when the device is located over the stoma, surround the stoma, the method including, when the model garment is worn by the ostimate, recording the unique marking of the or each area of the marked region overlying the ege or protuberances, so that the data provided indicates the boundary of the opening to be provided in the support garment.

8. A method according to claim 6 or claim 7 wherein the method includes connecting the opening locating device to the ostimate around the stoma.

9. A method according to any one of claims 6 to 8 wherein the opening locating device is sized to correspond to an annular flange attached to the ostimate and surrounding the stoma, the method including selecting an opening locating device of a size generally corresponding to the flange.

10. An opening locating device for locating an optimum position for an opening in an ostomy support garment on a model garment, the opening locating device including a peripheral formation generally corresponding in size to that of a flange to which in use an ostomy container is secured, and the device including a receiving part bounded by the peripheral formation, for receiving an ostimate's stoma, the device including a continuous edge or a plurality of protuberances which when the device is attached to the ostimate and the ostimate is wearing the model garment, is or are positioned around a boundary of the stoma, and indicate on the model garment the position of a boundary of the opening to be provided in the ostomy support garment.
